# EUROPÄISCHE PATENTSCHRIFT

(11) **EP 0 473 844 B1**
(45) Veröffentlichungstag und Bekanntmachung des Hinweises auf die Patenterteilung: **06.12.1995**
(21) Anmeldenummer: 90250220.2
(22) Anmeldetag: 04.09.1990
(51) Int. Cl.: A61K 31/13

(54) **Arzneimittel enthaltend eine Kombination von AZT und Amantadin für die Behandlung von AIDS**
Medicament containing a combination of AZT and amantadin for the treatment of AIDS
Médicament contenant une combinaison d'AZT et d'amantadine pour le traitement du SIDA

(43) Veröffentlichungstag der Anmeldung: 11.03.1992
(73) Patentinhaber: Lange, Werner, Prof., D-12163 Berlin (DE); Masihi, Kemuel Noel, Dr., D-12351 Berlin (DE)
(72) Erfinder: Lange, Werner, Prof., D-12163 Berlin (DE); Masihi, Kemuel Noel, Dr., D-12351 Berlin (DE)
(74) Vertreter: Wablat, Wolfgang, Dr.Dr.

(56) Entgegenhaltungen:
- EP-A- 0 135 312
- CHEMOTHERAPY, Band 38, Nr. 2, Januar 1990; M. ASANAKA et al., Seiten 249-255
- JOURNAL OF ANTIMICROBIAL CHEMOTHERAPY, Band 23, Suppl. A, Januar 1989, British Society for Antimicrobial Chemotherapy; J.S. OXFORD et al., Seiten 9-27
- INDEX NOMINUM 1987, 1987, Soc. Suisse de Pharmacie, Zurich (CH); "Amantadine (DCI prop.)"

## Beschreibung

Infektionen mit Retroviren gewinnen in Human- und Veterinärmedizin zunehmend an Interesse. Dabei steht das Human Immunodeficiency Virus (HIV) und das von ihm verursachte erworbene Immundefizienz-Syndrom (AIDS) aufgrund der zunehmenden Bedrohung für Gesundheit und Leben wachsender Teile der Bevölkerung im Vordergrund. Eine Darstellung der Situation findet sich beispielsweise in WHO: Weekly Epidemiological Record 64 (1989), Seiten 101-102.

Die Zahl von HIV-Infizierten und von an AIDS Erkrankten wächst ständig. Weltweit sind der Weltgesundheitsorganisation (WHO) derzeit mehr als 150 000 Erkrankungen bekannt. Etwa 40 % der Betroffenen sind verstorben. Die Zahlen der WHO sind als Mindestzahlen anzusehen, da mit einer hohen Dunkelziffer nicht gemeldeter Fälle gerechnet werden muß.

Die fortschreitende Beeinträchtigung der Immunabwehr bei Patienten mit AIDS führt zur Zunahme von opportunistischen Infektionen und Neoplasien. Von der Beherrschung opportunistischer Infektionen hängt die Überlebenszeit der Betroffenen entscheidend ab. Besser wäre es, die durch HIV bedingte Störung der Immunabwehr zu verhindern oder zu reduzieren. Deshalb ist die Suche nach Möglichkeiten der Verhinderung oder Beseitigung von Infektionen mit dem HIV von noch entscheidenderer Bedeutung und Ziel zahlreicher Forschungsvorhaben.

Die bisher bekannten Arzneimittel, die zur Behandlung des AIDS eingesetzt werden, verursachen erhebliche Nebenwirkungen, u.a. Leukopenien und Anämien, die bei AIDS-Patienten besonders häufig vorkommen und nicht selten zum Abbruch der Behandlung zwingen.

Darstellungen dieser Problematik finden sich insbesondere in den folgenden Literaturschriften:
a) Yeo, J.M.: Current and future trials with zidovudine, J. Infection 18 (1989) Suppl. 1, 93-96
b) Richman, D.D., Fischl, M.A., Grieco, MH. et al.: The toxicity of azidothymidine (AZT) in the treatment of patients with AIDS and AIDS-related complex New Engl. J. Med. 317 (1989) 192-197
c) Wainberg, M.A., Falutz, J., Fanning, M. Gill, J., Gelmon, K., Montaner J.S.G., O'Shaugnessy, M., Isoukas, Ch., Ruedy, J.:
   Cessation of Zidovudine Therapy May Lead to Increased Replication of HIV-1
   J. Amer.Med.Ass. 261 (1989) 865-866
d) Bach, M.C.: Failure of Zidovudine to Maintain Remission in Patients with AIDS
   New Engl. J. Med. 320 (1989) 594-595

### Weitere Literaturquellen:

e) CHEMOTHERAPY, Band 38, Nr. 2, 1990, Seiten 249 bis 255; M. ASANAKA et al.: "Screening of anti-HIV activities in existing drugs which are capable of long-term oral administration"
f) Journal of Antimicrobial Chemotherapy, Band 23, Suppl. A, Januar 1989, Seiten 9 bis 27, The British Society for Antimicrobial Chemotherapy; J.S. Oxford et al.: "Potential target sites for antiviral inhibitors of human immunodeficiency virus (HIV)"
g) Index Nominum 1987, Soc. Suisse de Pharmacie, Zürich, CH,; "Amantadine (DCI prop.)"
h) EP-A-0 135 312
i) Goodman and Gilman's the Pharmacological Basis of Therapeutics (1985), 7th Ed., p. 1232

berichten sowohl über Medikamente, die Amantadine als einzigen Wirkstoff oder in Kombination mit einer zusätzlichen antiviral wirksamen Substanz enthalten als auch über therapeutische Anwendungen der Amantadine, wie z. B. zur Behandlung von durch HIV hervorgerufene Erkrankungen.

Das einzige gegen Retroviren in vitro und in vivo wirksame Arzneimittel, Zidovudin (AZT), das für die Anwendung bei AIDS und AIDS Related Complex (ARC) zugelassen ist, besitzt ebenfalls eine Myelotoxizität. In letzter Zeit wurde mehrfach beschrieben, daß der durch hohe Nebenwirkungen erzwungene Abbruch der Therapie zu einer stärkeren Virusproduktion geführt hat als vor Beginn der Behandlung.

Es wäre daher falsch, derzeit zu behaupten, daß eine Chemotherapie oder -prophylaxe der HIV-Infektion und des AIDS zur Verfügung stehe. Deshalb laufen weltweit intensive Anstrengungen zur Entwicklung neuer antiviraler Substanzen und neuer Anwendungsformen mit verringerter Zytotoxizität. Ziel der Therapie muß die Eliminierung der Infektion, die Verhinderung der fortschreitenden Immunsuppression sowie die Verhütung opportunistischer Infektionen sein.

Aufgabe der Erfindung ist somit die Auffindung von Kombinationspräparaten, welche eine hohe antivirale Wirkung bei geringer Zytotoxizität und geringen Nebenwirkungen aufweisen.

Erfindungsgemäß wird dies durch die Verwendung von 1-Adamantanamin-hydrochlorid oder anderer Salze des 1-Adamantanamins, welche mit AZT kombiniert werden, erreicht.

Die aufgefundenen Präparate bzw. Kombinationen werden zur Behandlung von durch Retroviren hervorgerufene Erkrankungen, insbesondere AIDS, verwendet.

Gegenstand der Erfindung ist daher die Verwendung von Amantadin und AZT in einer synergistischen Kombination zur Herstellung eines Arzneimittels für die Behandlung von AIDS.

Erfindungsgemäß liegen die verwendeten Mengen von Amantadin und AZT unter den für eine isolierte Wirkung der Einzelsubstanzen benötigten Mengen.

Ein weiterer Gegenstand der Erfindung ist ein Arzneimittel, welches sich durch einen Gehalt an Amantadin und AZT in einer synergistischen Kombination und üblichen Trägerstoffen und/oder Zusätzen auszeichnet.

Die Erfindung ging von der Überlegung aus, daß es aufgrund der Besonderheiten der Virusvermehrung nicht möglich sein wird, eine Chemotherapie mit Monopräparaten zu entwickeln, die frei von Nebenwirkungen ist. Es muß deshalb versucht werden, durch Kombination synergistisch wirksamer Substanzen die Dosierung der Einzelkomponenten so niedrig wie möglich zu halten und damit ihre individuellen Nebenwirkungen zu reduzieren.

Überraschenderweise wurde beobachtet, daß Amantadin in vitro nach Infektion mit HIV-1 die Produktion von HIV-Antigenen in Monozyten und Lymphozyten signifikant verringern kann, wenn es in höheren Dosen allein oder in niedrigen Dosen mit AZT kombiniert angewandt wurde.

### VERSUCHSBESCHREIBUNG

### Beschreibung der Experimente

Für die Versuche wurden je eine Linie von menschlichen CD4-positiven monozytären Zellen und von menschlichen CD4-positiven lymphoblastoiden Zellen benutzt. Infektiöses HIV-1 wurde aus Überständen von infizierten Zellkulturen in der Phase der logarithmischen Vermehrung gewonnen. Nicht infizierte monozytäre oder lymphozytäre Zellen wurden für 2 Stunden mit 1 ml des Viruspools in Gegenwart von 2 »g Polybren inkubiert. Nach der Infektion wurden die Zellen pelletiert und in Kulturen zu 2 x 10⁵ Zellen pro Milliliter in supplementiertem Zellkulturmedium ausgesät. Die Bestimmung der Zellzahl erfolgte nach Färbung mit Trypanblau. In den Überständen der Kulturen wurde mit einem Enzymimmunoassay die Konzentration des HIV-Antigens p 24 bestimmt. Antigenkonzentrationen von unbehandelten und behandelten Kulturen wurden verglichen. Als Grad der antiviralen Aktivität der eingesetzten Substanzen wurde das Ausmaß der Verringerung der p24-Konzentration in Überständen infizierter Zellen gewertet.

### Ergebnisse

### 1. Wirkung von Amantadin und AZT in monozytären Zellen

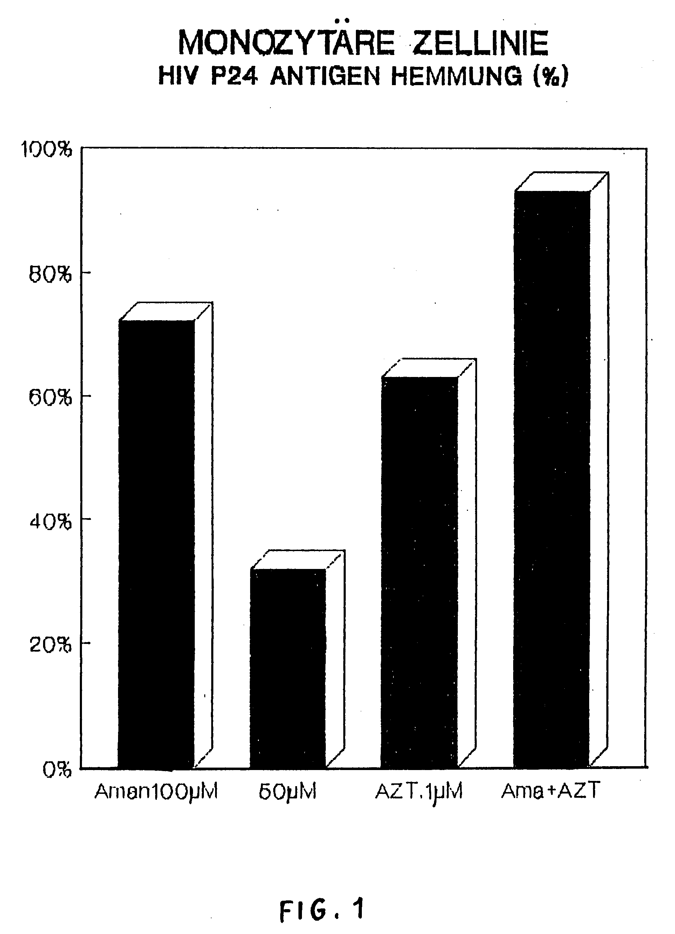

Die in Fig. 1 dargestellten Ergebnisse zeigen, daß 100 »M Amantadin die Produktion von p24-Antigen in HIV-1 infizierten monozytären Zellen um 72 % hemmen können. Sogar eine geringere Dosis von 50 »M Amantadin bewirkte noch eine Hemmung um 32 %. AZT hemmte in einer Dosis von 0,1 »M die Antigenproduktion um 62 %. Wenn man beide Substanzen kombiniert einsetzte, konnte eine Hemmung um 92-93 % erreicht werden.

### 2. Wirkung von Amantadin und AZT auf lymphozytäre Zellen

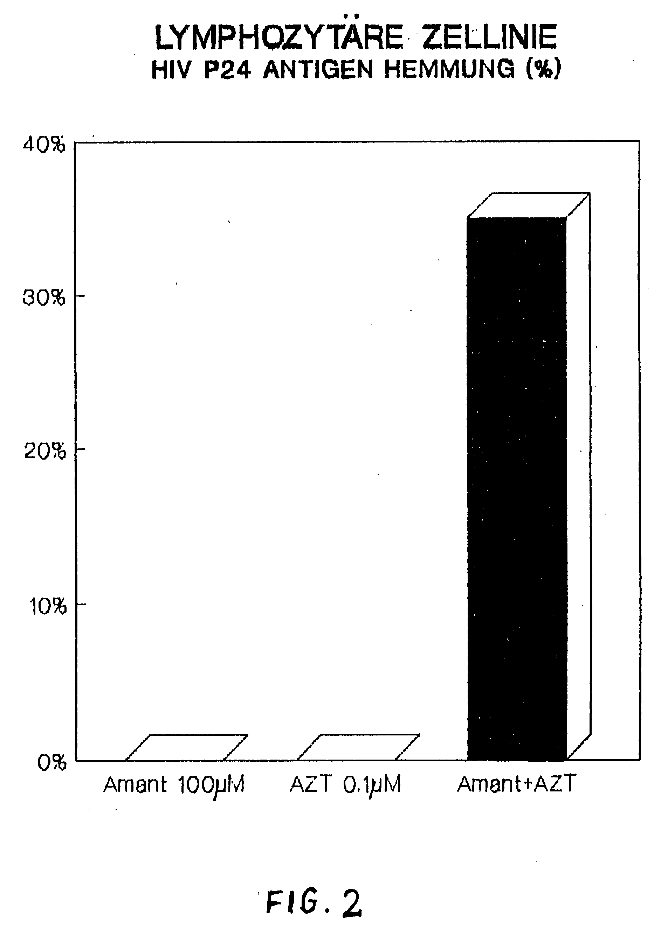

Die in Fig. 2 dargestellten Ergebnisse lassen erkennen, daß eine Kombination von 100 »M Amantadin und 0,1 »M AZT am Tag 3 nach Infektion die Produktion von p24-Antigen synergistisch hemmte. Am Tag 5 hemmten 0,01 »M AZT die Antigenproduktion um 76 %, in Kombination mit 100 uM Amantadin erreichte die Hemmwirkung 96 %. Diese Wirkung entspricht dem Effekt einer zehnfach höheren Dosis von AZT allein (Tab. 1).

**Tab.1**

| Wirkung von Amantadin auf die Produktion von p24-Antigen in HIV-infizierten H9-Zellen | | | |
|---|---|---|---|
| BEHANDLUNG | DOSIS (»M) | TAGE p.i. | PROZENT HEMMUNG |
| Amantadin | 100 | 3 | 0 |
| AZT | 0,1 | 3 | 0 |
| | 0,01 | 3 | 0 |
| Amantadin + AZT | 100 + 0,1 | 3 | 35 |
| Amantadin | 100 | 5 | 0 |
| AZT | 0,1 | 5 | 98 |
| | 0,01 | 5 | 76 |
| Amantadin + AZT | 100 + 0,01 | 5 | 96 |

Die Kombination von Amantadin mit AZT erlaubt eine wirksame Hemmung der Antigenproduktion von HIV, die als Maß für die Vermehrung des Virus gewertet wird. Dieser Effekt kann mit Konzentrationen erreicht werden, die unter den für eine isolierte Wirkung der Einzelsubstanzen benötigten Mengen liegt. Damit eröffnet sich die Möglichkeit, die bisher bei Anwendung von AZT beobachtete zytotoxische Wirkung in vitro und in vivo durch Reduzierung der notwendigen Dosierung von AZT zu verringern oder zu vermeiden. Die beobachtete Hemmwirkung der Kombination war deutlich größer als die der Einzelsubstanzen.

Bei beiden Substanzen handelt es sich um für die therapeutische Anwendung beim Menschen zugelassene Arzneimittel. Für beide sind Nebenwirkungen beschrieben, die ihre Anwendungsmöglichkeiten in den therapeutisch wirksamen Konzentrationen einschränken. Die synergistische Wirkung beider Substanzen gegen HIV-Infektionen eröffnet neue Möglichkeiten der Therapie bei reduzierten Nebenwirkungen.

Es wird auch davon ausgegangen, daß die für die Therapie mit AZT seit kurzem diskutierte Entwicklung einer "Resistenz" mit einer Kombination von AZT mit Amantadin zu umgehen ist.

Weitere Untersuchungen zeigten, daß die Anwendung von Amantadin allein oder in Kombination mit anderen antiviralen Substanzen auch bei Infektionen mit anderen Retroviren, z.B. mit dem Virus der menschlichen T-Zellen-Leukämie (HILV-1) und mit Retroviren bei Tieren, zu einer Hemmung der Virusvermehrung führt.

Demnach ist 1-Adamantanamin-hydrochlorid (Amantadin) allein und insbesondere in Kombination mit AZT in der Lage, in monozytären und lymphozytären Zellen die Vermehrung von Human Immunodeficiency Virus Typ 1 (HIV-1) signifikant zu hemmen (gezeigt durch Hemmung der Produktion von HIV-Antigen p24). Die Kombination von Amantadin mit AZT und anderen antiviralen Substanzen eröffnet völlig neue Möglichkeiten der Therapie von Infektionen mit HIV und anderen Retroviren des Menschen und der Tiere.

## Patentansprüche

1. Verwendung von Amantadin und AZT in einer synergistischen Kombination zur Herstellung eines Arzneimittels für die Behandlung von AIDS.

2. Verwendung von Amantadin und AZT nach Anspruch 1, dadurch gekennzeichnet, daß die verwendeten Mengen von Amantadin und AZT unter den für eine isolierte Wirkung der Einzelsubstanzen benötigten Mengen liegen.

3. Arzneimittel, gekennzeichnet durch einen Gehalt an Amantadin und AZT in einer synergistischen Kombination und üblichen Trägerstoffen, Hilfsstoffen und/oder Zusätzen.

## Claims

1. Application of amantadine and AZT in a synergistic combination for the production of a medicament for the treatment of AIDS.

2. Application of amantadine and AZT according to claim 1, characterized in that the amount of amantadine and AZT used is below the amount necessary for an isolated effect of the individual substances.

3. Medicament, characterized by a content of amantadine and AZT in a synergistic combination and customary vehicle substances, adjuvants and/or additives.

## Revendications

1. Utilisation d'amantadine et d'AZT en association à effet de synergie, pour la fabrication d'un médicament destiné au traitement du SIDA.

2. Utilisation d'amantadine et d'AZT selon la revendication 1, caractérisée en ce que les quantités d'amantadine et d'AZT utilisées correspondent à des quantités inférieures aux quantités nécessaires pour l'action isolée d'une substance prise séparément.

3. Médicament comprenant de l'amantadine et de l'AZT en association à effet de synergie et des véhicules, adjuvants et/ou additifs usuels.
